# EUROPÄISCHE PATENTSCHRIFT

(11) **EP 2 322 232 B1**
(45) Veröffentlichungstag und Bekanntmachung des Hinweises auf die Patenterteilung: **13.09.2017**
(21) Anmeldenummer: 10075735.0
(22) Anmeldetag: 12.11.2010
(51) Int. Cl.: A61L 15/28

(54) **VERFAHREN ZUR HERSTELLUNG EINER SCHICHTFÖRMIGEN WUNDAUFLAGE**
Method for producing a layered wound dressing
Procédé destiné à la fabrication d'un pansement en forme de couche

(30) Priorität: 12.11.2009 DE 102009053305
(43) Veröffentlichungstag der Anmeldung: 18.05.2011
(73) Patentinhaber: Medichema GmbH, 09112 Chemnitz (DE)
(72) Erfinder: Pallaske, Frank, 09355 Gersdorf (DE)
(74) Vertreter: Neumann, Günter

(56) Entgegenhaltungen:
- EP-A1- 0 431 479
- EP-A1- 1 022 031
- DE-A1-102006 020 498
- US-A1- 2004 243 043
- US-A1- 2006 134 185
- YUDANOVA T N ET AL: "Modern wound dressings: Manufacturing and properties", PHARMACEUTICAL CHEMISTRY JOURNAL, KLUWER ACADEMIC PUBLISHERS-CONSULTANTS BUREAU, NE, Bd. 40, Nr. 2, 1. Februar 2006 (2006-02-01), Seiten 85-92, XP019405075, ISSN: 1573-9031, DOI: 10.1007/S11094-006-0065-Z

## Beschreibung

Die Erfindung betrifft ein Verfahren zur Herstellung einer schichtförmigen Wundauflage sowie eine nach dem Verfahren hergestellte Wundauflage. Derartige Wundauflagen finden insbesondere als blutstillendes und wundversiegelndes Mittel sowie zur Verbindung von getrennten Körpergewebsbereichen Verwendung. Wundauflagen zu dem genannten Zweck und Verfahren zu deren Herstellung sind hinlänglich bekannt.
Aus der EP 0 815 879 A2 geht ein Verfahren hervor, das sich auf die Herstellung einer bioabsorbierbaren Einheit bezieht und das Lösen eines Cellulosederivates, das aus der Gruppe, bestehend aus Methylcellulose, Carboxymethylcellulose oder Celluloseacetat, besteht, in Wasser und das anschließende Oxidieren des Celluslosederivates umfasst. Diese Einheit soll in Form eines Filmes, eines Gels, eines Pulvers, einer faserigen Matte oder eines Schwammes zur Verfügung gestellt werden können.
Die EP 1 378 255 B1 beschreibt ebenfalls ein Verfahren zur Herstellung eines einschichtigen hämostatischen Wundverbandes. Es umfasst die Bereitstellung einer Lösung mit einem darin im wesentlichen gelösten wasserlöslichen oder wasserquellbaren biokompatiblen Polymers, ferner die Bereitstellung eines aus Fasern eines biokompatiblen Polymers bestehenden und Flexibilität, Festigkeit und Porosität aufweisenden Stoffes und das Eintauchen dieses Stoffes in die Lösung mit anschließendem Gefriertrocknen zum Erhalt einer porösen polymeren Matrix.

Einen einschichtigen absorbierbaren Verbundstoff aus einer unlöslichen Kollagenmatrix mit Chlorhexidin und Calziumalginat zur Verwendung in der Behandlung von Wurzelhauterkrankungen des Zahnes offenbart die DE 694 28 312 T2. Vorgewaschenes faserartiges Kollagen wird in sauberem deionisiertem pyrogen-freiem Wasser suspendiert und zu einer feinfaserigen Suspension homogenisiert und anschließend angesäuert, um eine aufgequollene, faserartige Dispersion zu erhalten. Diese wird mit einer homogenisierten Suspension aus Calziumalginatfasern und Chlorhexidindigluconat gemischt, die erhaltene Mischung danach entgast und bei Raumtemperatur getrocknet. Der getrocknete Stoff kann danach in Streifen geschnitten werden.

Ein ebenfalls einschichtiges selbsthaftendes resorbierbares Hämostyptikum offenbart die DE 103 18 802 A1. Mindestens ein sich in Lösung oder im Gelzustand befindliches Polymer, vorzugsweise Polysaccharid, wird durch Gefriertrocknung in eine feste trockene Form überführt.

Ein Verfahren zur Herstellung einer mehrschichtigen Kollagenmatrix zur Geweberekonstruktion geht aus der DE 600 18 480 T2 hervor. Zwei Lagen einer porösen Matrix aus biokompatiblem Kollagen werden bei gleichmäßigem und gleichzeitigem Einsetzen von mechanischem Druck und erhöhter Temperatur zu einer Mehrschichtlage zusammengefügt.

Ein weiteres Verfahren zur Herstellung einer mehrschichtigen Wundauflage auf Basis von Collagen offenbart die DE 697 24 243 T2. Die einzelnen Schichten können Thrombin oder Vorläufer von Thrombin enthalten. Als Thrombinstabilisator dienen unter anderem Polysaccharide. Die Haftung der Schichten erfolgt beispielsweise durch gleichzeitiges oder sequentielles Gefrieren, Lyophilisieren, durch Vernetzen oder Verkleben.

Eine schichtförmige Wundauflage mit mindestens einem bioverträglichen Trägermaterial und mindestens einem Polysaccharid und ein Verfahren zu deren Herstellung beschreibt die DE 10 2006 020 498 A1. Mindestens eine Dispersion des Trägermaterials in einem flüssigen Dispersionsmittel wird durch Abkühlung und Einfrierung unter Ausbildung mindestens einer festen Trägerschicht verfestigt, auf die anschließend das Polysaccharid aufgebracht wird, das durch erneute Abkühlung zu einer festen Schicht des Polysaccharides auf der Trägerschicht verfestigt wird. Unter Entfernung des Dispersionsmittels sollen die beiden Schichten miteinander verbunden werden.

Bei diesem Verfahren, welches mindestens ein zweimaliges Einfrieren erfordert, besteht die Gefahr, dass die erste eingefrorene Schicht wieder auftaut. Durch die in ihrer Bewegung gehemmten Moleküle der eingefrorenen Schicht ist darüber hinaus die Haftung der beiden Schichten nur schwach ausgeprägt. So kann eine verstärkte Aufnahme von Wundsekret dazu führen, dass sich die einzelnen Schichten wieder voneinander lösen. Außerdem ist der Einfrierprozess sehr umständlich, da das Produkt nach dem ersten Einfriervorgang aus der Kammer des Einfrierapparates entnommen werden muss und sich dabei die Kammer und das Produkt wieder erwärmt. Anschließend erfolgen erneut das Runterkühlen der Kammer und das Einfrieren der bereits schon einmal eingefrorenen Schicht mit der darauf befindlichen noch nicht eingefrorenen Schicht.

Aus EP 0 431 479 A1 geht eine Wundauflage hervor, die hauptsächlich aus Kollagen und Chitin besteht. Die Herstellung dieser Wundauflage setzt ein Chitinvlies als Trägermaterial voraus. Die Chitinschicht und die Kollagenschicht sind entweder mittels eines biokompatiblen Klebers oder durch Nähen miteinander verbunden. Die Verbindung kann auch durch Auflegen des Chitinvlieses auf die Kollagensuspension und anschließendes Gefriertrocknen hergestellt werden.
Eine Wundauflage, deren Herstellung ebenfalls eine Trägerschicht erfordert offenbart US 2004/24303043 A1. Sie besteht aus einer Chitosanschicht und einer Chitinschicht. Eine als Trägermaterial fungierende Chitin-Vliesmatte wird auf eine Chitosan-Lösung gelegt. Daran schließen sich eine Gefriertrocknung und ein energieintensiver thermischer Pressvorgang an.

Aufgabe der Erfindung ist es, ein verbessertes Verfahren für die Herstellung einer mehrschichtigen Wundauflage bereitzustellen, das die Vorzüge der Verwendung biokompatibler Materialien beibehält, einfacher und kostengünstiger zu handhaben ist und ohne eine separat herzustellende Trägerschicht eine höhere Stabilität und durchgehende Verbindung zwischen den schichtförmig ausgebildeten Stoffen ermöglicht, sowie die entsprechende mehrschichtige Wundauflage bereitzustellen, die eine für eine effektive Sekretaufnahme vorteilhafte Porenstruktur des Polysaccharides aufweist. Erfindungsgemäß wird die Aufgabe durch ein Verfahren zur Herstellung einer mehrschichtigen Wundauflage mit mindestens einem bioverträglichen Material und mindestens einem Polysaccharid, gelöst, das die Schritte des Anspruchs 1 umfasst. Als Polysaccharid wird Alginat, Amylose, Amylopektin, Cellulose, Chitosan, Chondroitinsulfat, Dextran, Hyaluronsäure oder Stärke verwendet. Als synthetisches Polymer wird ein Polyvinylalkohol oder Polyethylenglykol od er als Polypeptid Kollagen oder Kollagen mit einem Zusatz von Elastin oder ein Gemisch von diesen Materialien verwendet und das flüssige Dispersionsmittel enthält bis zu 30 Gew. % Wasser.
Nach einer weiteren Ausführungsform der Erfindung wird als organische wasserhaltige Flüssigkeit Ethanol, Isopropanol oder Glycerin verwendet.
Vorzugsweise wird für das Verfahren eine Polysacchariddispersion mit einer Konzentration von bis zu 40 Gew. % des Polysaccharides, bezogen auf das Gesamtgewicht der Polysacchariddispersion und/oder eine Suspension aus Kollagen in einer Konzentration von bis zu 1,5 Gew. %, bezogen auf das Gesamtgewicht der Suspension eingesetzt.
Eine Ausführungsform sieht vor, dass die Wundauflage mit einer Dicke des schichtförmigen Polysaccharides von 0,5 mm bis 5 mm und/oder mit einer Dicke des schichtförmigen bioverträglichem Material von 0,02 mm bis 5 mm hergestellt wird und/oder zur Kenntlichmachung der Wundauflage das Polysaccharid und/oder das bioverträgliche Material eingefärbt werden.
Vorteilhafterweise enthält die Wundauflage antimikrobielle und/oder entzündungshemmende Wirkstoffe und/oder ist die Oberfläche der Wundauflage modifiziert geglättet oder strukturiert wellen- oder waffelförmig ausgebildet.
Gemäß einer weiteren besonders bevorzugten Ausführungsform des Verfahrens wird die Wundauflage unter Verwendung eines Aldehydes oder mehrerer Aldehyde als Vernetzungsmittel und/oder von 1,2-Propandiol als Weichmacher und/oder von Kollagen bovinen, equinen und/oder porcinen Ursprungs hergestellt.
Eine weitere Ausführungsform der Erfindung sieht vor, dass auf die eingefrorene Wundauflage bestehend aus dem schichtförmigen bioverträglichen Material und dem Polysaccharid eine neue Schicht aufgebracht und diese dann unter Gefriertrocknung zu einer dreischichtigen Wundauflage ausgebildet wird.
Die Aufgabe wird des Weiteren durch eine Wundauflage mit den Merkmalen des Anspruches 13 gelöst.

Unter dem Begriff "bioverträglich" im Sinne der vorliegenden Erfindung soll die Eigenschaft eines Materials verstanden werden, das bei Kontakt mit Körpergeweben keine unerwünschten Nebenwirkungen hervorruft.

Mit der Erfindung verbindet sich der besondere Vorzug, dass eine mehrschichtige Wundauflage in nur einem einzigen Gefriertrocknungsprozess hergestellt werden kann.
Durch die Erfindung ist es des Weiteren möglich, bei gegebener Bioverträglichkeit und Resorbierbarkeit die Stabilität der Wundauflage in besonderem Maße zu verbessern. Dies wird insbesondere durch die wesentlich stärkere Haftung der gegenüberliegenden Oberflächen des bioverträglichen Materials und des Polysaccharides auf Grund des nur einmaligen Gefriervorgangs der beiden Materialien erreicht. Die gesonderte Ausbildung einer Trägerschicht erweist sich bei dem erfindungsgemäßen Verfahren im Unterschied zum Stand der Technik nicht mehr als erforderlich. Des Weiteren zeichnet sich die erfindungsgemäß hergestellte Wundauflage durch eine bessere und steuerbare Wundsekretaufnahmekapazität aus. Diese wird durch das verwendete Polysaccharid und die Struktur des Polysaccharides in der Wundauflage beeinflusst. Die Wundsekretaufnahmekapazität kann gezielt durch das Dispersionsmittel der Polysacchariddispersion, das verwendete Polysaccharid und das Quellen der verfestigten Polysacchariddispersion eingestellt werden. Ebenso ist es möglich, Wundauflagen je nach Anwendungsfall mit sehr unterschiedlichen Flächengewichten im Bereich von 0,01 g/cm² und 0,15 g/cm² herzustellen. Des Weiteren kann die Dichte und die Stärke des bioverträglichen Materials und des Polysaccharides gezielt beeinflusst werden.
Der Einfluss von Polysacchariden auf die Geschwindigkeit der Exsudataufnahme ist bekannt. Je mehr Polysaccharid für die Wundauflage verwendet wird, desto mehr kann diese Wundsekrete aufnehmen. Jedoch werden aber auch die Wundauflagen zunehmend schwerer und härter. Es besteht daher das Bedürfnis, die Wundsekretaufnahmekapazität ohne wesentliche Erhöhung des Flächengewichtes der Wundauflage zu verbessern.
Das verwendete Dispersionsmittel beeinflusst vor allem die Geschwindigkeit der Exsudataufnahme durch die Wundauflage. Leicht verdampfbare Dispersionsmittel entweichen im Rahmen der Trocknung aus der Wundauflage, dadurch bleiben Poren, welche für eine schnelle Sekretaufnahme von Bedeutung sind, erhalten. Dagegen führt eine hohe Konzentration schwer verdampfbarer Dispersionsmittel zur Verdichtung und damit zur Zerstörung der Polysaccharid- sowie der Matrix des bioverträglichen Materials. Dadurch wird Porenstruktur des Polysaccharides insoweit negativ beeinflusst, dass die Wundauflage nur noch relativ langsam Sekrete aufnehmen kann.
Durch die definierte Zugabe von Wasser für den Quellvorgang lassen sich die Dicke und die Porenstruktur der Polysaccharidmatrix gezielt beeinflussen. Das ist mit dem Vorzug verbunden, dass die Geschwindigkeit der Exsudataufnahme auf diese Weise gesteuert und für die typischen Anwendungsfälle der Wundauflage ausgerichtet werden kann.

Ist die Quellung des Polysaccharides nahezu abgeschlossen und wird auf dieses eine Polypeptidsuspension, bevorzugt eine Kollagensuspension, aufgebracht, so wird sich die Dicke des flächenmäßig vorliegenden Polysaccharides nur in geringen Toleranzen ändern. Erfolgt dagegen der Quellvorgang des Polysaccharides nur unter Zugabe von verhältnismäßig wenig Wasser und wird auf das verfestigte Polysaccharid die Polypeptidsuspension gegeben, so wird aus dieser das Wasser für den weiteren Quellvorgang entzogen. Dadurch verdichtet sich das Polypeptid. Es entstehen Wundauflagen mit einem relativ dicken schichtförmigen Polysaccharid und einem verhältnismäßig sehr dünn schichtförmig ausgebildeten Polypeptid, welches nur noch in geringem Maße Exsudat aufnehmen kann und so die Wundauflage nach außen abdichtet.

Im Folgenden soll die Erfindung an Ausführungsbeispielen näher erläutert werden.

### Beispiel 1:

Es wurden 11 g Carboxylmethylcellulose und 18 ml Glycerin mit einem Wassergehalt von 15 % zu einer Dispersion gemischt und in Gießschalen gegeben. Durch die Aufnahme des Wassers aus der Dispersionslösung in das Polysaccharid verfestigte sich die Dispersion. Auf diese wurden 75 ml Reinstwasser gegeben um ein Aufquellen zu erreichen. Die Quellzeit betrug 1 h. Anschließend wurden auf das gequollene Gel 100 ml einer Kollagensuspension gegeben. Als Kollagensuspension wurde eine aus Schweinehaut nach bekanntem Verfahren, der Extraktion des Kollagens und dem Quellen im Wasser bei pH zwischen 3 bis 5, gewonnene Suspension mit einem Gehalt von 1 Gew. % Kollagen verwendet. Beide Materialien wurden danach abgekühlt und zusammen eingefroren und gefriergetrocknet. Unter Entfernung des Wassers verbanden sich die beiden Materialien zu einer flächenmäßig als Vlies ausgebildeten schichtförmigen Wundauflage. Sie ist weich, verform- und schneidbar. Die Wundauflage besitzt ein Flächengewicht von 0,125 g/cm². Sowohl die Carboxylmethylcellulose als auch das Kollagen besitzen jeweils eine Dicke von 1,5 mm bis 2,5 mm.

Je nach dem vorgesehenen Verwendungszweck kann die Wundauflage mit Wirkstoffen versehen und gemäß den Anforderungen in der gewünschten Größe geschnitten, sterilisiert, konfektioniert und aseptisch abgefüllt werden.

Die so hergestellt Wundauflage kann bis zu 100 ml Exsudat aufnehmen.

### Beispiel 2:

Es wurden 3 g Carboxymethylcellulose mit 10 ml 96%igem Ethanol zu einer Dispersion gemischt. Nach dem Verdunsten des Ethanols entstand das verfestigte Polysaccharid. Auf das Polysaccharid wurden 60 ml Reinstwasser gegeben. Anschließend wurde, wie im Beispiel 1 beschrieben, eine Kollagensuspension auf das nach einer Stunde vollständig gequollene Polysaccharid gegeben. Die weiteren Behandlungsschritte erfolgten wie im Beispiel 1 beschrieben. Die Wundauflage ist ebenfalls weich, verform- und schneidbar. Die Wundauflage besitzt ein Flächengewicht von 0,016 g/cm². Sowohl die Carboxylmethylcellulose als auch das Kollagen besitzen jeweils eine Dicke von 1,5 mm bis 2,5 mm.

Das schichtförmige Polysaccharid der Wundauflage kann kleine Volumen an Exsudat ca. 10 ml in weniger als 10s aufnehmen.

Sofern eine Kennzeichnung der erfindungsgemäßen Wundauflage entsprechend der vorgesehenen Verwendung erfolgen soll, so kann das Polysaccharid oder das Kollagen eingefärbt werden, vorzugsweise mittels Riboflavin

## Patentansprüche

1. Verfahren zur Herstellung einer schichtförmigen Wundauflage mit mindestens einem bioverträglichen Material und mindestens einem Polysaccharid, umfassend die Schritte
- Bereitstellung mindestens einer Dispersion aus Alginat, Amylose, Amylopektin, Cellulose Chitosan, Chondroitinsulfat, Dextran, Hyaloronsäure oder Stärke als Polysaccharid in einer wässrigen organischen Flüssigkeit mit einem Wassergehalt bis zu 30 Gew.% als flüssiges Dispersionsmittel;
- Verfestigung der Dispersion unter schichtförmiger Ausbildung des Polysaccharides durch Aufnahme von Wasser aus dem flüssigen Dispersionsmittel und/oder Aufnahme des Dispersionsmittels in das Polysaccharid und/oder durch Verdunstung des Dispersionsmittels;
- durch Zufuhr von Wasser Aufquellen des verfestigten Polysaccharides unter Bildung eines Gels sowie Einstellung dessen Dicke und Porenstruktur;
- Aufbringen einer Suspension oder Lösung eines Saccharides, Lipides, synthetischen Polymers oder eines Polypeptides oder eines synthetischen Polymers als bioverträgliches Material auf das gequollene Gel,
- gleichzeitiges Abkühlen und Gefriertrocknen des Gels mit der aufgebrachten Suspension oder Lösung des bioverträglichen Materials unter Ausbildung einer festen Verbindung zwischen den beiden Schichten.

2. Verfahren nach Anspruch 1, **dadurch gekennzeichnet, dass** als sythetisches Polymer ein Polyvinylalkohol oder Polyethylenglykol oder als Polypeptid Kollagen oder Kollagen mit einem Zusatz von Elastin oder ein Gemisch von diesen Materialien verwendet wird.

3. Verfahren nach Anspruch 1, **dadurch gekennzeichnet, da**ss als organische wasserhaltige Flüssigkeit Ethanol, Isopropanol oder Glycerin oder ein Gemisch dieser Stoffe verwendet wird.

4. Verfahren nach mindestens einem der Ansprüche 2 bis 3, **dadurch gekennzeichnet, dass** die Polysacchariddispersion mit einer Konzentration von bis zu 40 Gew. % des Polysaccharides, bezogen auf das Gesamtgewicht der Polysacchariddispersion und/oder die Suspension aus Kollagen in einer Konzentration von bis zu 1,5 Gew. %, bezogen auf das Gesamtgewicht der Suspension bereitgestellt wird.

5. Verfahren nach mindestens einem der Ansprüche 1 bis 4, **dadurch gekennzeichnet, dass** die Wundauflage mit einer Dicke des schichtförmigen Polysaccharides von 0,5 mm bis 5 mm und/oder mit einer Dicke des schichtförmigen bioverträglichem Material von 0,02 mm bis 5 mm hergestellt wird.

6. Verfahren nach mindestens einem der Ansprüche 1 bis 5, **dadurch gekennzeichnet, dass** zur Kenntlichmachung der Wundauflage das Polysaccharid und/oder das bioverträgliche Material eingefärbt werden.

7. Verfahren nach Anspruch 6, **dadurch gekennzeichnet, dass** zur Einfärbung Riboflavin verwendet wird.

8. Verfahren nach mindestens einem der Ansprüche 1 bis 7, **dadurch gekennzeichnet, dass** die Wundauflage antimikrobielle und/oder entzündungshemmende Wirkstoffe enthält und/oder die Oberfläche der Wundauflage modifiziert geglättet oder strukturiert wellen- oder waffelförmig ausgebildet wird.

9. Verfahren nach mindestens einem der Ansprüche 1 bis 8, **dadurch gekennzeichnet, dass** die Wundauflage unter Verwendung eines Vernetzungsmittels hergestellt wird.

10. Verfahren nach Anspruch 9, **dadurch gekennzeichnet, dass** als Vernetzungsmittel ein Aldehyd oder mehrerer Aldehyde und/oder 1,2-Propandiol als ein Weichmacher verwendet werden.

11. Verfahren nach Anspruch 2, **dadurch gekennzeichnet, dass** als Kollagen ein Kollagen bovinen, equinen und/oder porcinen Ursprungs eingesetzt wird.

12. Verfahren nach mindestens einem der Ansprüche 1 bis 11, **dadurch gekennzeichnet, dass** auf die eingefrorene Wundauflage bestehend aus dem schichtförmigen bioverträglichen Material und dem Polysaccharid eine neue Schicht aufgebracht und diese dann unter Gefriertrocknung zu einer dreischichtigen Wundauflage ausgebildet wird.

13. Schichtförmige Wundauflage bestehend aus mindestens einem bioverträglichen Material und mindestens einem Polysaccharid, hergestellt nach dem Verfahren gemäß den Ansprüchen 1 bis 12.

## Claims

1. A method for producing a layered wound dressing with at least a biocompatible material and at least a polysaccharide comprising the following steps
- providing at least a dispersion ou of alginate, amylose, amylopectin, cellulose, chitosan, chondroitin sulphate, dextran, hyaluronic acid or starch as a polysaccharide in an aqueous, organic liquid with a water content up to 30 wt% as a liquid dispersing agent;
- solidifying the dispersion by a layered formation of the polysaccharide through the absorption of water from the liquid dispersing agent and/ or the absorption of the dispersing agent into the polysaccharide and/ or by evaporation of the dispersing agent;
- by the supply of water, swelling of the solidified polysaccharide by forming a gel as well as adjustment of its thickness and pore structure;
- applying a suspension or solution of a saccharide, lipid, synthetic polymer or a polypeptide or a synthetic polymer as a biocompatible material onto the swollen gel,
- simultaneous cooling and freeze-drying of the gel with the applied suspension or solution of the biocompatible material by forming a strong bonding between the two layers.

2. The method according to claim 1, **characterised in that** as a synthetic polymer, a polyvinyl alcohol or polyethylene glycol, or as a polypeptide, collagen or collagen with the addition of elastin or a mixture of these materials is used.

3. The method according to claim 1, **characterised in that** as an organic, aqueous liquid, ethanol, isopropanol or glycerin or a mixture of these substances is used.

4. The method according to at least any one of claims 2 to 3, **characterised in that** the polysaccharide dispersion is provided with a concentration of up to 40 wt% of the polysaccharide based on the total weight of the polysaccharide dispersion and/or the suspension made out of collagen is provided in a concentration of up to 1.5 wt% based on the total weight of the suspension.

5. The method according to at least any one of claims 1 to 4, **characterised in that** the wound dressing is produced with a thickness of the layered polysaccharide of 0,5 mm to 5 mm and/or with a thickness of the layered, biocompatible material of 0.02 mm to 5 mm.

6. The method according to at least any one of claims 1 to 5, **characterised in that** for identification of the wound dressing the polysaccharide and/or the biocompatible material are dyed.

7. The method according to claim 6, **characterised in that** riboflavin is used for dying.

8. The method according to at least any one of claims 1 to 7, **characterised in that** the wound dressing contains antimicrobial and/or anti-inflammatory agents and/or the surface of the wound dressing is formed in a modified and smoothed way or textured in an undulated or waffle-type manner.

9. The method according to at least any one of claims 1 to 8, **characterised in that** the wound dressing is produced by using a cross-linking agent.

10. The method according to claim 9, **characterised in that** as a cross-linking agent, one aldehyde or several aldehydes are used and/or 1,2-propanediol is used as a softening agent.

11. The method according to claim 2, **characterised in that** as a collagen, a collagen of bovine, equine and/or porcine origin are used.

12. The method according to at least any one of claims 1 to 11, **characterised in that** onto the frozen wound dressing consisting of a layered biocompatible material and the polysaccharide, a new layer is applied which layer is then formed into a three-layered wound dressing.

13. A layered wound dressing consisting of at least a biocompatible material and at least a polysaccharide produced by the method according to claims 1 to 12.

## Revendications

1. Procédé destiné à la fabrication d'un pansement en forme de couche avec au moins un matériau biocompatible et au moins un polysaccharide comportant les étapes suivantes
- prévoir au moins une dispersion de l'alginate, de l'amylose, de l'amylopectine, de cellulose, de chitosan, de sulfate de chondroïtine, de dextrane, de l'acide hyaluronique ou de l'amidon en tant que polysaccharide dans un liquide aqueux organique avec une teneur en eau jusqu'à 30 % en poids en tant qu'agent de dispersion liquide;
- solidifier la dispersion par formation en forme de couche du polysaccharide en absorbant de l'eau à partir de l'agent de dispersion liquide et/ou en absorbant de l'agent de dispersion dans le polysaccharide et/ou par évaporation de l'agent de dispersion;
- par apport d'eau, gonflement du polysaccharide solidifié en formant un gel ainsi qu'ajustement de son épaisseur et de sa structure poreuse;
- appliquer une suspension ou une solution d'un saccharide, d'un lipide, d'un polymère synthétique ou d'un polypeptide ou d'un polymère synthétique en tant que matériau biocompatible sur le gel gonflé,
- refroidissement et lyophilisation simultané du gel avec la suspension ou la solution appliquée du matériau biocompatible sous formation d'une liaison étroite entre les deux couches.

2. Procédé selon la revendication 1, **caractérisé en ce qu**'en tant que polymère synthétique, un alcool polyvinylique ou un polyéthylène glycol est utilisé ou en tant que polypeptide, du collagène ou du collagène avec addition de l'élastine ou un mélange de ces matériaux est utilisé.

3. Procédé selon la revendication 1, **caractérisé en ce qu**'en tant que liquide aqueux organique de l'éthanol, de l'isopropanol ou de la glycérine ou un mélange de ces substances est utilisé.

4. Procédé selon au moins l'une quelconque des revendications 2 à 3, **caractérisé en ce que** la dispersion du polysaccharide est fourni avec une concentration de jusqu'au 40 % en poids du polysaccharide par rapport au poids total de la dispersion du polysaccharide et/ou que la suspension de collagène est fourni avec une concentration de jusqu'au 1,5 % en poids par rapport au poids total de la suspension.

5. Procédé selon au moins l'une quelconque des revendications 1 à 4, **caractérisé en ce que** le pansement est fabriqué avec une épaisseur du polysaccharide en forme de couche de 0,5 mm à 5 mm et/ou avec une épaisseur du matériau biocompatible en forme de couche de 0,02 mm à 5 mm.

6. Procédé selon au moins l'une quelconque des revendications 1 à 5 , **caractérisé en ce que** pour l'identification du pansement, le polysaccharide et/ou le matériau biocompatible sont colorés.

7. Procédé selon la revendication 6, **caractérisé en ce que** de la riboflavine est utilisée pour la coloration.

8. Procédé selon au moins l'une quelconque des revendications 1 à 7, **caractérisé en ce que** le pansement contient des agents antimicrobiens et/ou des agents anti-inflammatoires et/ou que la surface du pansement est formée de manière modifiée et lisse ou texturée de manière ondulée ou gaufrée.

9. Procédé selon au moins l'une quelconque des revendications 1 à 8, **caractérisé en ce que** le pansement est fabriqué en utilisant un agent de réticulation.

10. Procédé selon la revendication 9, **caractérisé en ce qu**'en tant qu'agent de réticulation un aldéhyde ou plusieurs aldéhydes sont utilisés et/ou en tant que plastifiant 1,2-propanediol est utilisé.

11. Procédé selon la revendication 2, **caractérisé en ce qu**'en tant que collagène un collagène de provenance bovine, équine et/ou porcine est utilisé.

12. Procédé selon au moins l'une quelconque des revendications 1 à 11, **caractérisé en ce que** sur le pansement gelé constitué par un matériau biocompatible en forme de couche et le polysaccharide une nouvelle couche est appliquée et que cette couche est ensuite formée en un pansement en trois couches par lyophilisation.

13. Pansement en forme de couche constitué par au moins un matériau biocompatible et au moins un polysaccharide fabriqué conformément au procédé selon les revendications 1 à 12.
